(19) Europäisches Patentamt
European Patent Office
Office européen des brevets

(11) Publication number: 0 106 588 B1

(12) # EUROPEAN PATENT SPECIFICATION

(45) Date of publication of patent specification: 30.04.86

(51) Int. Cl.[4]: C 07 C 121/75, C 09 K 19/20, C 09 K 19/30

(21) Application number: 83305765.6

(22) Date of filing: 27.09.83

(54) 2-Cyano-4-halogenophenyl esters.

(30) Priority: 27.09.82 JP 168174/82
19.11.82 JP 203492/82
22.01.83 JP 8896/83
24.01.83 JP 9646/83
02.03.83 JP 34224/83
16.03.83 JP 43596/83
09.04.83 JP 62417/83
01.06.83 JP 97284/83

(43) Date of publication of application: 25.04.84 Bulletin 84/17

(45) Publication of the grant of the patent: 30.04.86 Bulletin 86/18

(84) Designated Contracting States: CH DE GB LI

(56) References cited:
DE-A-3 001 423
GB-A-2 070 593
GB-A-2 072 214
US-A-4 198 312

(73) Proprietor: Chisso Corporation
6-32, Nakanoshima 3-chome Kita-ku
Osaka-shi Osaka-fu (JP)

(72) Inventor: Sugimori, Shigeru
2493-10, Fujisawa
Fujisawashi Kanagawaken (JP)
Inventor: Isoyama, Toyoshiro
10-3, Otsutomocho Kanazawaku
Yokohamashi Kanagawaken (JP)
Inventor: Kojima, Tetsuhiko
10-3, Otsutomocho Kanazawaku
Yokohamashi Kanagawaken (JP)
Inventor: Goto, Yasuyuki
10-3, Otsutomocho Kanazawaku
Yokohamashi Kanagawaken (JP)

(74) Representative: Ruffles, Graham Keith et al
MARKS & CLERK 57-60 Lincoln's Inn Fields
London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 106 588 B1

**Description**

This invention relates to novel 2-cyano-4-halogenophenyl esters as well as to liquid crystal compositions containing the esters.

Liquid crystal display elements utilize the optical anisotropy and dielectric anisotropy of liquid crystal substances, and are classified into various types such as the TN (twisted, nematic) type, DS (dynamic scattering) type, guest-host type, DAP type, according to their display mode. While the particular properties required of liquid crystal substances for the respective uses are different, it is generally the case in any mode that the liquid crystal substances have to be stable to heat, air, light, etc. and also for preference exhibit the liquid crystal phase in temperature ranges as broad as possible, around room temperature. At present, however, no single compound is available which alone satisfies such conditions, and it is the state of the art that resort has to be made to a mixture of liquid crystal compounds or a mixture of one or more of these compounds and compounds which themselves are non-liquid crystalline but which when combined with liquid crystal compounds can form a liquid crystal composition.

Recently, guest-host type liquid crystal display elements have come to the fore as a mode of colour liquid crystal display. For such elements, mixtures of liquid crystals and dyestuffs are used, and for positive type guest-host type display elements, liquid crystals having a negative dielectric anisotropy are used. Thus, as the constituents of such liquid crystal mixtures, liquid crystal compounds which have various specific properties, a good compatibility and a negative dielectric anisotropy have been required.

The object of the present invention is to provide novel compounds which are useful as constituents of liquid crystal compositions exhibiting a negative dielectric anisotropy.

The present invention resides in 2-cyano-4-halogenophenyl esters expressed by the general formula:

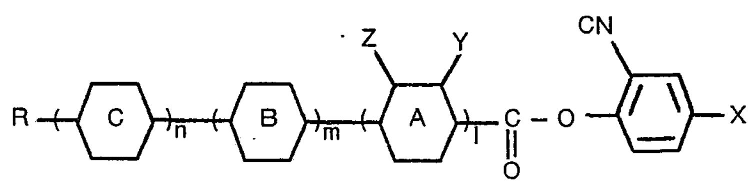

(I)

wherein R represents a hydrogen atom, or an alkyl group or an alkoxy group of 1 to 10 carbon atoms; the rings

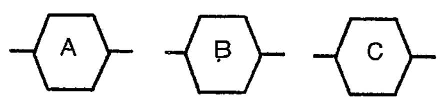

each represent

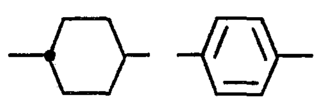

*l, m and n* each represent 0 or 1 and the total of l+m+n is 1, 2 or 3; X represents F or Cl; and Y and Z each represent a hydrogen atom or F or Cl.

German Offenlegungsschrift 3001423 describes compounds of the general formula

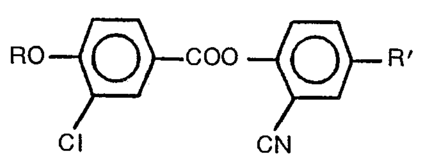

in which R and R′ are straight chain alkyl of up to 8 carbon atoms. The compounds are for use in liquid crystal compositions, but suffer from a relatively high viscosity, among other defects.

GB 2070593 describes halogenated ester derivatives expressed by the general formula

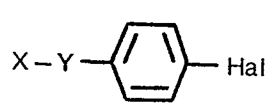

wherein X represents a group selected from the group consisting of

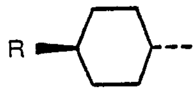 , 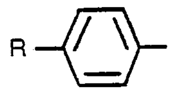 , 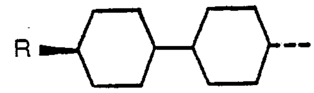 ,

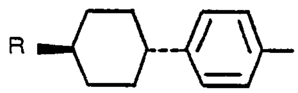 , 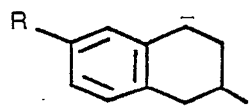 and 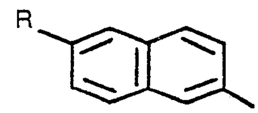

R represents alkyl or alkoxy of 1 to 15 carbons and Y is oxycarbonyl or carbonyloxy. These compounds have a positive dielectric anisotropy.

The present invention further resides in liquid crystal compositions containing at least one of the novel esters of formula (I), and display elements utilizing the compositions.

Examples of the compounds of the formula (I) can be concretely classified into the following four types: compounds expressed by the general formula

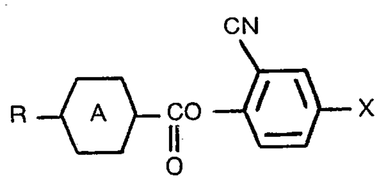

(II)

wherein R, the ring

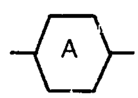

and X are each as defined above; compounds expressed by the general formula

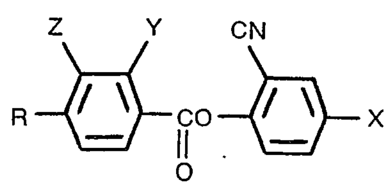

(III)

wherein R, X, Y and Z are each as defined above: compounds expressed by the general formula

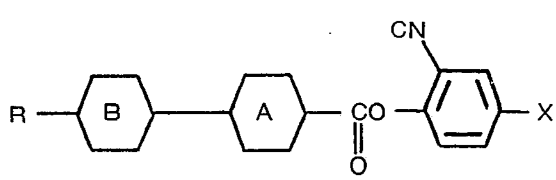

(IV)

wherein R, the rings

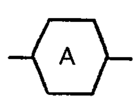 and 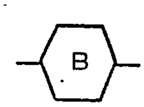

and X are each as defined above; and compounds expressed by the general formula

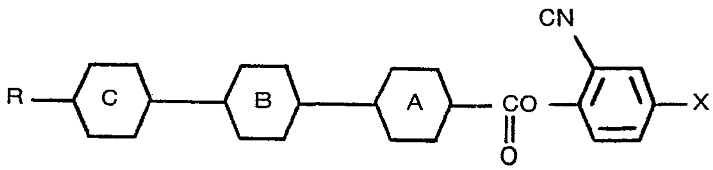

wherein R, the rings

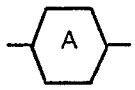,
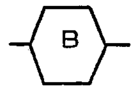
and
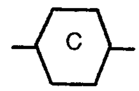

and X are each as defined above.

Examples of the group R include methyl, ethyl, propyl, butyl, pentyl, hexyl, heptyl and octyl groups; methoxy, ethoxy, propoxy, butoxy, pentoxy, hexoxy, heptoxy and octoxy groups; as well as a hydrogen atom.

Among the compounds of this invention, those of the formulae (IV) and (V) are liquid crystal compounds exhibiting a nematic liquid phase up to considerably high temperatures, and have a dielectric anisotropy value of about −4, whereas compounds of the formulae (II) and (III) do not exhibit a liquid crystal phase, but, when blended with liquid crystal compounds, exhibit the same effectiveness. The above-mentioned compounds of the formulae (IV), (V), (II) and (III) are stable to heat, light, moisture, air, etc., and when they are blended with other nematic liquid crystals such as those of the cyclohexanecarboxylic acid phenyl ester group, benzoic acid phenyl ester group, phenylmetadioxane group, phenylpyrimidine group, etc., it is possible to obtain liquid crystal compositions having a negative dielectric anisotropy and usable for guest-host type display elements. Further they are usable as an additive in liquid crystal compositions for two-frequent addressing scheme as well as multiplex drive which have recently been noted. In general, the compounds (I) are useful in liquid crystal display elements.

The present invention also provides a process for the preparation of the esters of the present invention. In accordance with the present process, a 2-cyano-4-halophenol is reacted with a carboxylic acid corresponding to the desired end-product, preferably using the acid in the form of a reactive derivative such as the acid chloride. The process is preferably carried out in the presence of pyridine or other suitable organic base.

The starting 2-cyano-4-halophenols can be prepared by dehydrating the oxime of a 5-halosalicylaldehyde with acetic acid anhydride or other suitable dehydrating agent, as shown by the following equation:

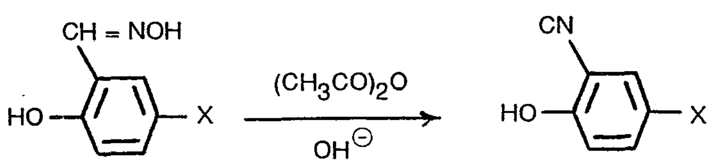


The parent carboxylic acids with one or two rings are generally known, and the synthesis may be modified to produce other acids of the same kind. As for the carboxylic acids having three rings, the acids of the type (A), (C), (E) and (G) shown below are generally known, and the syntheses may be modified to produce other acids of the same kind. Furthermore, these three-ring acids can be reduced with metallic sodium in isoamyl alcohol, or using other suitable conditions, to give the acids of the type (B), (D), (F) and (H):

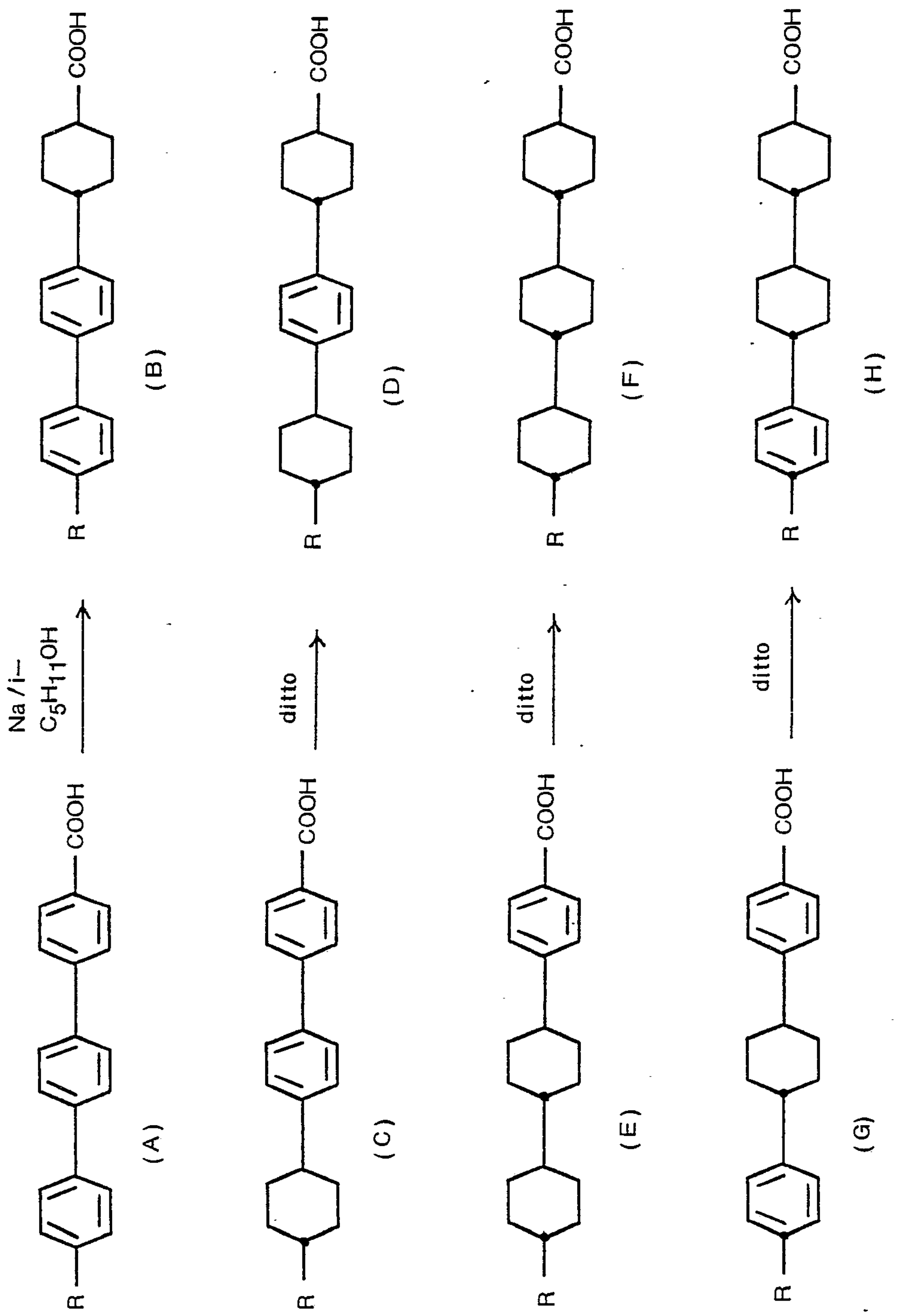


(A) (B)

(C) (D)

(E) (F)

(G) (H)

These carboxylic acids can be converted in a conventional manner into the corresponding acid chlorides or other reactive derivative, which are then reacted with the 2-cyano-4-substituted phenols to obtain the desired compounds of the formula (I). This reaction is illustrated by the following chemical equation:

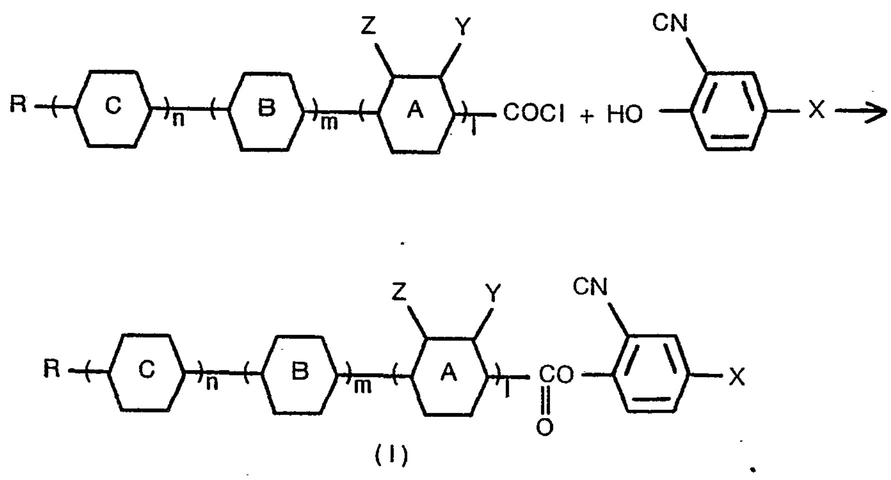

(I)

The present invention will be further described in detail by way of Examples.

Example 1

Preparation of trans-4-(trans-4-propylcyclohexyl)cyclohexanecarboxylic acid 2-cyano-4-fluorophenyl ester, a compound of the formula (IV) wherein R is $C_3H_7$

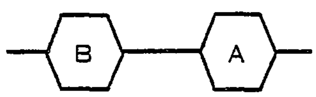

is

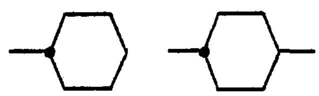

and X is F

(1) Preparation of 2-cyano-4-fluorophenyl

5-Fluorosalicylaldehyde oxime (20.2 g, 0.13 mol) was dissolved in acetic anhydride (100 ml) and refluxed for 5 hours. After completion of the reaction, acetic anhydride was distilled off under reduced pressure followed by adding to the remaining oily substance, a solution of KOH (20 g) dissolved in water (100 ml) and ethanol (100 ml), warming the mixture at 80°C for 2 hours, allowing it to cool down to room temperature, adding 6N hydrochloric acid (50 ml) and water (200 ml) to deposit crystals, which were then filtered off and recrystallized from methanol (30 ml) to obtain needle crystals (15.6 g) having a melting point of 121—122°C.

(2) Esterification

2-Cyano-4-fluorophenol (1.5 g, 0.011 mol) obtained in the step (1) was dissolved in dried pyridine (20 ml), and to the resulting solution was added a solution obtained by dissolving trans-4-(trans-4'-propylcyclohexyl)cyclohexanecarboxylic acid chloride (3.0 g, 0.011 mol) in dry toluene (20 ml), followed by reaction at 60°C for 3 hours. After completion of the reaction, the reaction product was poured in water (100 ml), followed by separating the toluene layer, washing with 6N hydrochloric acid, 2N NaOH solution and then with water, drying the toluene layer over anhydrous sodium sulfate, distilling off toluene and recrystallizing the remaining crystals from ethyl acetate (10 ml) to obtain the objective trans-4-(trans-4'-propylcyclohexyl)cyclohexanecarboxylic acid-2-cyano-4-fluorophenyl ester (2.7 g, yield 68%), C—N point: 108.7°C, N—I point: 150.0°C.

Examples 2—65

Example 1 was repeated except that trans-4-(trans-4'-propylcyclohexyl)cyclohexanecarboxylic acid chloride in Example 1 was converted into the corresponding various carboxylic acid chlorides, and besides 2-cyano-4-fluorophenol, 2-cyano-4-chlorophenol in place of 2-cyano-4-fluorophenol was used, to obtain other compounds of the formula (I). The phase transition points of these compounds are shown in Table 1 (compounds of the formula (IV)), Table 2 (compounds of the formula (II)), Table 3 (compounds of the formula (III)) and Table 4 (compounds of the formula (V)).

TABLE 1

Phase transition points of compounds of formula (IV)

| Example | In formula IV | | | | Phase transit on point (°C) | |
|---|---|---|---|---|---|---|
| | R | 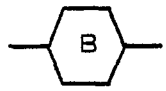 | 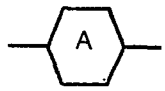 | X | C–N point (m.p.) | N–I point |
| 1 | $C_3H_7$ | 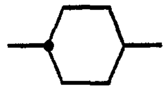 | 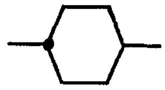 | F | 108.7 | 150.0 |
| 2 | $C_2H_5$ | ,, | ,, | F | 109.8 | 117.0 |
| 3 | $C_4H_9$ | ,, | ,, | F | 114.8 | 148.6 |
| 4 | $C_5H_{11}$ | ,, | ,, | F | 117.6 | 152.1 |
| 5 | $C_7H_{15}$ | ,, | ,, | F | 109.6 | 143.4 |
| 6 | $C_2H_5$ | ,, | 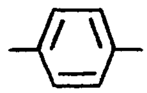 | F | 81.3 | (53.9) |
| 7 | $C_3H_7$ | ,, | ,, | F | 93.5 | (92.3) |
| 8 | $C_4H_9$ | ,, | ,, | F | 67.2 | 93.0 |
| 9 | $C_5H_{11}$ | ,, | ,, | F | 85.6 | 105.6 |
| 10 | $C_7H_{15}$ | ,, | ,, | F | 82.9 | 104.0 |
| 11 | $C_5H_{11}$ | 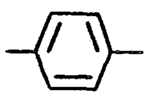 | 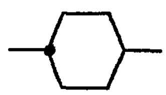 | F | 76.7 | (28.8) |
| 12 | $C_2H_5$ | 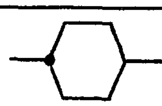 | 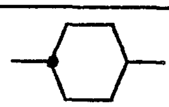 | Cl | 91.3 | 149.9 |
| 13 | $C_3H_7$ | ,, | ,, | Cl | 103.4 | 180.4 |
| 14 | $C_4H_9$ | ,, | ,, | Cl | 90.6 | 176.7 |
| 15 | $C_5H_{11}$ | ,, | ,, | Cl | 101.8 | 178.7 |
| 16 | $C_5H_{11}$ | 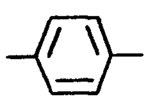 | ,, | Cl | 71.8 | (60.6) |

TABLE 1 (Continued)

Phase transition points of compounds of formula (IV)

| Example | In formula IV | | | | Phase transition point (°C) | |
|---|---|---|---|---|---|---|
| | R | 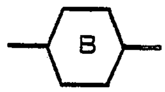 | 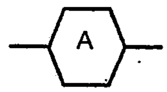 | X | C–N point (m.p.) | N–I point |
| 17 | $C_3H_7$ | 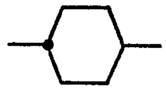 | 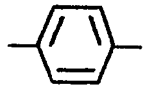 | Cl | 113.6 | 127.3 |
| 18 | $C_5H_{11}$ | ,, | ,, | Cl | 100.0 | 129.0 |
| 19 | $C_7H_{15}$ | ,, | ,, | Cl | 98.1 | 127.0 |

Note: Numeral values in the parenthese represent monotropic liquid crystals.

TABLE 2

Phase transition points of compounds of formula (II)

| Example | In formula (II) | | | Phase transition point (°C) |
|---|---|---|---|---|
| | R | 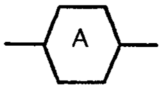 | X | C–I point (m.p.) |
| 20 | $C_2H_5$ | 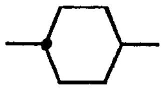 | F | 71.0 |
| 21 | $C_3H_7$ | ,, | F | 72.8 |
| 22 | $C_4H_9$ | ,, | F | 80.9 |
| 23 | $C_5H_{11}$ | ,, | F | 72.1 |
| 24 | $C_6H_{13}$ | ,, | F | 86.3 |
| 25 | $C_7H_{15}$ | ,, | F | 79.6 |
| 26 | $C_3H_7$ | 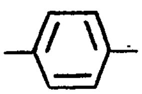 | F | 58.6 |
| 27 | $C_4H_9$ | ,, | F | 28.4 |
| 28 | $C_5H_{11}$ | ,, | F | 49.0 |
| 29 | $C_7H_{15}$ | ,, | F | 48.6 |
| 30 | $C_2H_5$ | 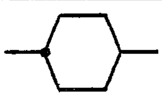 | Cl | 45.5 |
| 31 | $C_3H_7$ | ,, | Cl | 54.9 |
| 32 | $C_4H_9$ | ,, | Cl | 55.6 |
| 33 | $C_5H_{11}$ | ,, | Cl | 55.9 |
| 34 | $C_6H_{13}$ | ,, | Cl | 67.0 |
| 35 | $C_7H_{15}$ | ,, | Cl | 63.4 |
| 36 | $C_8H_{17}$ | ,, | Cl | 72.3 |

TABLE 2 (Continued)

Phase transition points of compounds of formula (II)

| Example | In formula (II) | | | Phase transition point (°C) |
|---|---|---|---|---|
| | R | 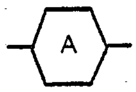 | X | C–I point (m.p.) |
| 37 | $C_2H_5$ | 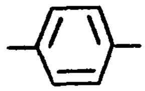 | Cl | 79.6 |
| 38 | $C_4H_9$ | ,, | Cl | 35.9 |
| 39 | $C_5H_{11}$ | ,, | Cl | 51.6 |
| 40 | $C_7H_{15}$ | ,, | Cl | 62.0 |
| 41 | $C_8H_{17}$ | ,, | Cl | 60.1 |
| 42 | $C_2H_5O$ | 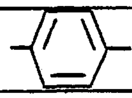 | Cl | 177.5 |
| 43 | $C_3H_7O$ | ,, | Cl | 108.9 |
| 44 | $C_4H_9O$ | ,, | Cl | 85.9 |
| 45 | $C_5H_{11}O$ | ,, | Cl | 70.8 |
| 46 | $C_6H_{13}O$ | ,, | Cl | 90.0 |
| 47 | $C_7H_{15}O$ | ,, | Cl | 74.7 |

TABLE 3

Phase transition points of compounds of formula (III)

| Example | In formula (III) | | | | Phase transition point (°C) |
|---|---|---|---|---|---|
| | R | X | Z | Y | C–I point (°C) |
| 48 | $C_5H_{11}O$ | F | Cl | H | 122.0 |
| 49 | $CH_3$ | F | F | H | 135.2 |
| 50 | $C_5H_{11}O$ | Cl | Cl | H | 120.0 |
| 51 | $CH_3$ | Cl | F | H | 152.8 |
| 52 | $CH_3O$ | Cl | Cl | H | 186.8 |
| 53 | $C_5H_{11}O$ | Cl | H | Cl | 96.1 |
| 54 | $C_3H_7O$ | Cl | H | Cl | 117.2 |
| 55 | $C_5H_{11}O$ | F | H | Cl | 101.9 |

TABLE 4

Phase transition points of compounds of formula (V)

| Example | In formula (V) | | | | | Phase transition point (°C) | | |
|---|---|---|---|---|---|---|---|---|
| | R | 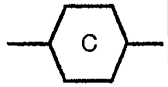 | 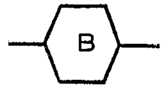 | 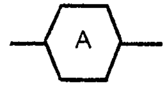 | X | C–S point or C–N point | S–N point | N–I point |
| 56 | $C_5H_{11}$ | 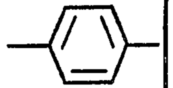 | 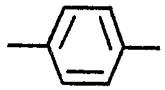 | 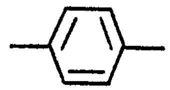 | F | 167.0 | 173.4 | 274.5 |
| 57 | $C_5H_{11}$ | ,, | ,, | ,, | Cl | 157.6 | 159.2 | 284.1 |
| 58 | $C_5H_{11}$ | 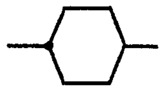 | 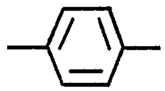 | 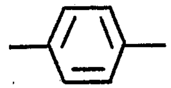 | F | 93.8 | – | 266.0 |
| 59 | $C_5H_{11}$ | ,, | ,, | ,, | Cl | 105.2 | – | 274.5 |
| 60 | $C_5H_{11}$ | 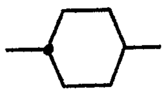 | 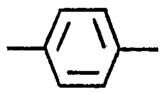 | 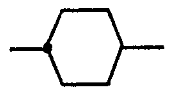 | F | 118.0 | – | 231.6 |
| 61 | $C_5H_{11}$ | ,, | ,, | ,, | Cl | 106.8 | – | 214.0 |
| 62 | $C_3H_7$ | 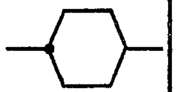 | 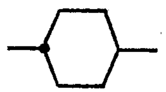 | 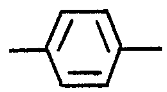 | F | 112.9 | – | 203.7 |
| 63 | $C_3H_7$ | ,, | ,, | ,, | Cl | 130.1 | – | 247.0 |
| 64 | $C_3H_7$ | 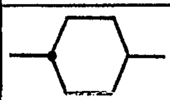 | 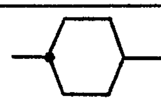 | 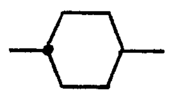 | F | 190.4 | – | 246.0 |
| 65 | $C_3H_7$ | ,, | ,, | ,, | Cl | 160/5 | – | 267.0 |

Example 66 (Use example 1)

A nematic liquid crystal composition of esters A consisting of

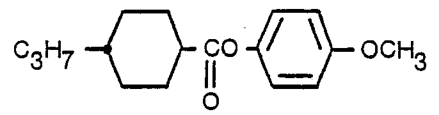 10.4% by weight

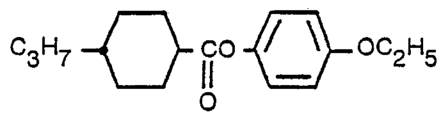 10.3% ,, ,,

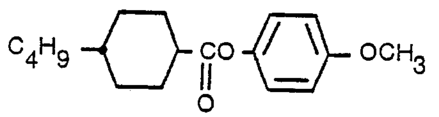 21.1% ,, ,,

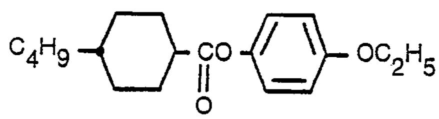 19.8% ,, ,,

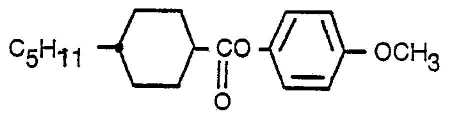 21.0% ,, ,, and

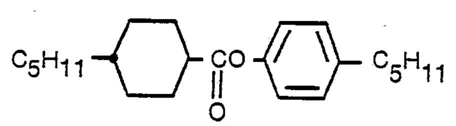 17.4% ,, ,,

has a N—I point of 62.8°C, a $\Delta\varepsilon$ of −1.0 and a viscosity at 20°C of 18.5 cp. A commercially available dyestuff (G-224, made by Mercke Co.) (1%) was added to the composition A, and the mixture was sealed in a cell to prepare a liquid crystal cell of guest-host type, followed by measuring its threshold voltage to give a value of 3.80 V. Next, to the above liquid crystal composition A (75 parts by weight) were added trans-4-propylcyclohexanecarboxylic acid-2-cyano-4-fluorophenyl ester (compound of Example 21 of the present invention) (5 parts by weight), trans-4-butylcyclohexanecarboxylic acid-2-cyano-4-fluorophenyl ester (compound of Example 22) (5 parts by weight) aand trans-4-pentylcyclohexanecarboxylic acid-2-cyano-4-fluorophenyl ester (compound of Example 23) (5 parts by weight) to prepare a liquid crystal composition, which had a N—I point of 47°C, a $\Delta\varepsilon$ of −2.7 and a density at 20°C of 24.7 cp. To this composition was added the same dyestuff G-224 (1%) as above to prepare a liquid crystal cell of guest-host type, the threshold voltage of which was then measured to give a notably reduced value of 3.10.

Example 67 (Use example 2)

To the nematic liquid crystal composition A (80 parts by weight) werre added trans-4-heptylcyclohexanecarboxylic acid-2-cyano-4-chlorophenyl ester of Example 35 (a compound of the present invention) (10 parts by weight) and trans-4-(trans-4′-propylcyclohexyl)cyclohexanecarboxylic acid-2-cyano-4-chlorophenyl ester of Example 13 (10 parts by weight) to prepare a liquid crystal composition, which had a N—I point of 68°C, a $\Delta\varepsilon$ of −4.0 and a viscosity at 20°C of 25.5 cp. The same dyestuff G-224 as above (1%) was added to the composition. Using the resulting composition, a guest-host type liquid crystal cell was prepared, and its threshold voltage was measured to give a notably reduced value of 3.20 V.

Example 68 (Use example 3)

2-Chloro-4-pentyloxybenzoic acid-2-cyano-4-chlorophenyl ester of Example 50 (a compound of the present invention) (10 parts by weight) was blended with the above-mentioned nematic liquid crystal composition A (90 parts by weight) to prepare a liquid crystal composition, which had a N—I point of 34.5°C, a $\Delta\varepsilon$ of −1.2 and a viscosity at 20°C of 28.5 cp. The same dyestuff G-224 as above (1%) was added to the composition. Using the resulting composition, a guest-host type liquid crystal cell was prepared, and its threshold voltage was measured to give a notably reduced value of 3.26 V.

Example 69 (Use example 4)

An ester nematic liquid crystal composition B consisting of

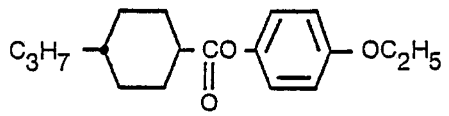

10% by weight

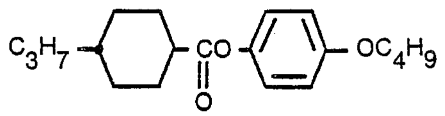

16% by weight

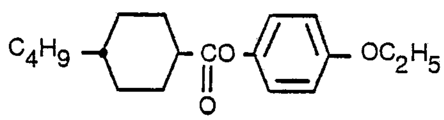

12% by weight

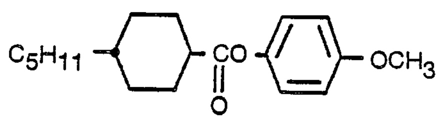

12% by weight

and

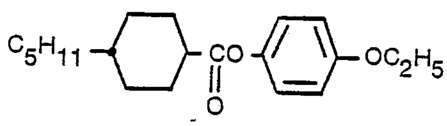

8% by weight

has a N—I point of 74.5°C, a dielectric anisotropy value of Δε of −1.4 and a viscosity at 20°C of 20.3 cp. To this composition was added the same dyestuff G-224 as above (1%) and the mixture was sealed in a cell to prepare a guest-host cell, followed by measuring its threshold voltage to give a value of 3.7 V. Next, trans-4-(trans-4′-propylcyclohexyl)cyclohexanecarboxylic acid-2-cyano-4-fluorophenyl ester of Example 1 (a compound of the present invention) (5 parts by weight), trans-4-(trans-4′-butylcyclohexyl)cyclohexanecarboxylic acid-2-cyano-4-fluorophenyl ester of Example 3 (5 parts by weight and trans-4-(trans-4′-pentylcyclohexyl)cyclohexanecarboxylic acid-2-cyano-4-fluorophenyl ester of Example 4 (5 parts by weight) were added to the above liquid crystal composition B (75 parts by weight) to prepare a liquid crystal composition, which had a N—I point of 79.2°C, a dielectric anisotropy Δε of −2.4 and a viscosity at 20°C of 27 cp. To this composition was added the same dyestuff G-224 as above (1%), followed by measuring its threshold voltage to give a notably reduced value of 2.9 V.

Example 70 (Use example 5)

To the above-mentioned nematic liquid crystal composition A (85 parts by weight) were added 4-[trans-4-(trans-4-propylcyclohexyl)cyclohexyl]benzoic acid-2-cyano-4-chlorophenyl ester of Example 63 (10 parts by weight) and trans-4″-propyl-trans-octadecahydro-p-terphenyl-trans-4-carboxylic acid-2-cyano-4-chlorophenyl ester of Example 65 (5 parts by weight) to prepare a liquid crystal composition, which had a N—I point of 32.3°C, a Δε of −1.2 and a viscosity at 20°C of 30.0 cp. To the composition was added the same dyestuff G-224 (1% by weight) as above, to prepare a guest-host cell the threshold voltage of which was measured to give a notably reduced value of 3.20 V.

Example 71 (Use example 6)

To the above-mentioned nematic liquid crystal composition A (85 parts by weight) were added 4-(trans-4-pentylcyclohexyl)biphenyl-4′-carboxylic acid-2-cyano-4-fluorophenyl ester of Example 62 (a compound of the present invention) (5 parts by weight), trans-4-[4-(trans-4-pentylcyclohexyl)phenyl]cylohexanecarboxylic acid-2-cyano-4-fluorophenyl ester of Example 60 (5 parts by weight) and trans-4-[4-(trans-4-pentylcyclohexyl)phenyl]cyclohexanecarboxylic acid-2-cyano-4-chlorophenyl ester of Example 63 (5 parts by weight), to prepare a liquid crystal composition, which had a N—I point of 83.4°C, a Δε of −1.2 and a viscosity at 20°C of 30.3 cp. To this composition was added the same dyestuff G-224 as above (1 wt.%) and the mixture was sealed in the same cell as above to prepare a guest-host type liquid crystal cell the threshold voltage of which was measured to give a notably reduced value of 3.26 V.

**Claims**

1. A 2-cyano-4-halogenophenyl ester expressed by the general formula

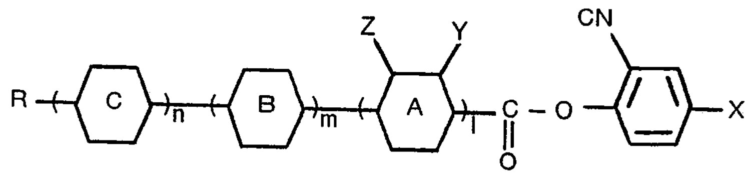

(I)

wherein R represents a hydrogen atom or an alkyl group or an alkoxy group of 1 to 10 carbon atoms; the rings

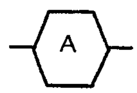
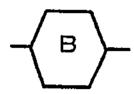
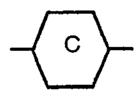

each represent

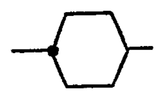

or

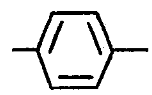

*l*, *m* and *n* each represent 0 or 1 and the total of l + m + n is 1, 2 or 3; X represents F or Cl; and Y and Z each represent a hydrogen atom or F or Cl.

2. A 2-cyano-4-halogenophenyl ester expressed by the general formula

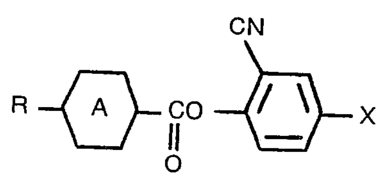

(II)

wherein R, the ring

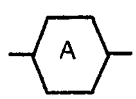

and X are each as defined in claim 1.

3. A 2-cyano-4-halogenophenyl ester expressed by the general formula

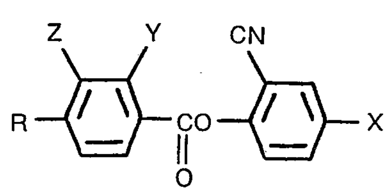

(III)

wherein R, X, Y and Z are each as defined in claim 1.

4. A 2-cyano-4-halogenophenyl ester expressed by the general formula

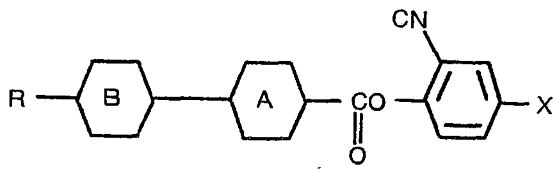

(IV)

wherein R, the rings

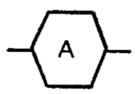

and

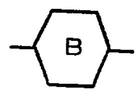

and X are each as defined in claim 1.

5. A 2-cyano-4-halogenophenyl ester expressed by the general formula

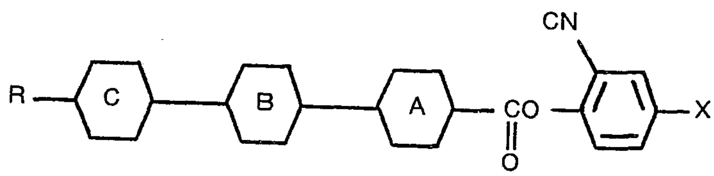

(V)

wherein R, the rings

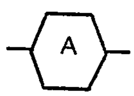

,

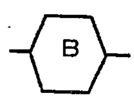

and

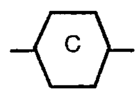

and x are each as defined in claim 1.

6. A liquid crystal composition containing at least one ester according to any preceding claim.

7. A liquid crystal display element which contains a composition according to claim 6.

8. A process for producing an ester according to claim 1, which comprises reacting a 2-cyano-4-halophenol of general formula

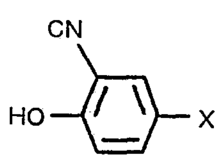

where X is as defined, with a carboxylic acid of formula

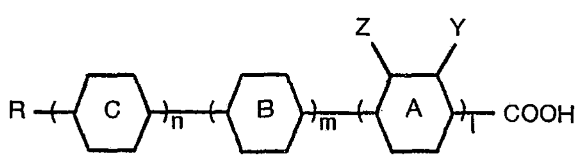

where R, A, B, C, X, Y, Z, l, m and n are as defined in claim 1.

**Patentansprüche**

1. 2-Cyano-4-halogenphenylester mit der allgemeinen Formel

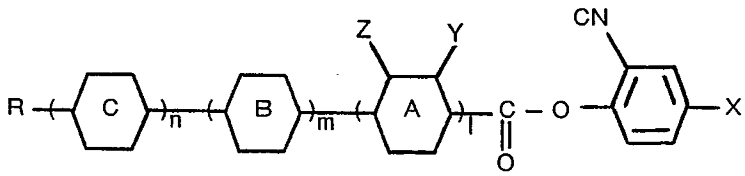

(I)

worin R ein Wasserstoffatom oder eine Alkylgruppe oder eine Alkoxygruppe mit 1 bis 10 Kohlenstoffatomen darstellt, die Ringe

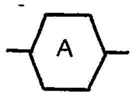

,

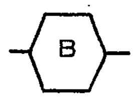

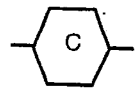

jeweils sind

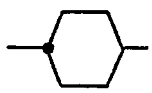

oder

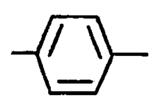

*l*, *m* and *n* jeweils 0 oder 1 sind und l + m + n zusammen 1, 2, oder 3; X, F oder Cl darstellt und Y und Z jeweils ein Wasserstoffatom oder F oder Cl darstellen.

2. 2-Cyano-4-halogenphenylester mit der allgemeinen Formel

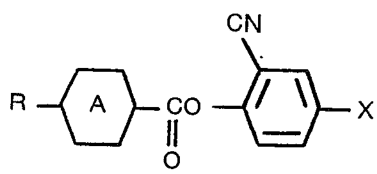

(II)

worin R, der Ring

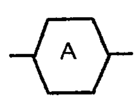

und X jeweils so definiert sind wie im Anspruch 1.

3. 2-Cyano-4-halogenphenylester mit der allgemeinen Formel

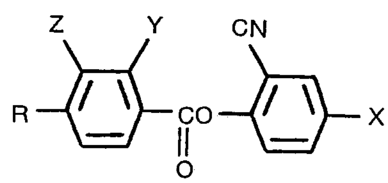

(III)

worin R, X, Y und Z jeweils so definiert sind wie im Anspruch 1.

4. 2-Cyano-4-halogenphenylester mit der allgemeinen Formel

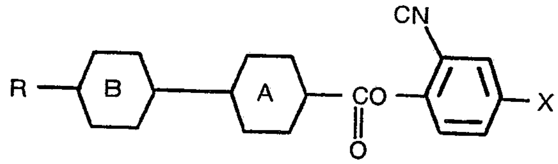

(IV)

worin R, die Ringe

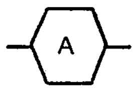 und 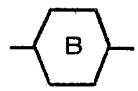

sowix X jeweils so definiert sind wie im Anspruch 1.

5. 2-Cyano-4-halogenphenylester mit der allgemeinen Formel

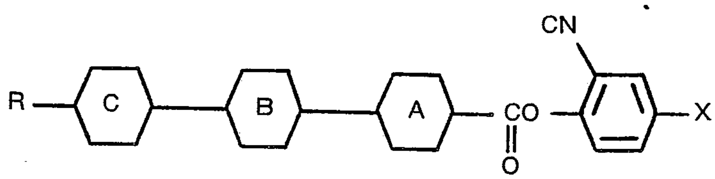

(V)

worin R, die Ringe

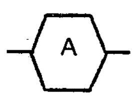 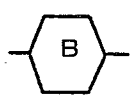 und 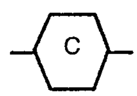

sowix X jeweils so definiert sind wie im Anspruch 1.

6. Flüssigkristallzusammensetzung, welche wenigstens einen Ester nach einem der vorstehenden Ansprüche enthält.

7. Flüssigkristallanzeigeelement, welches eine Zusammensetzung nach dem Anspruch 6 enthält.

8. Verfahren zur Herstellung eines Esters nach Anspruch 1, bei welchem ein 2-Cyano-4-halogenphenol der allgemeinen Formel

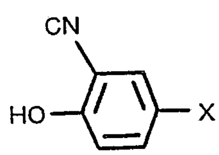

worin X so ist wid definiert mit einer Carbonsäure der Formel

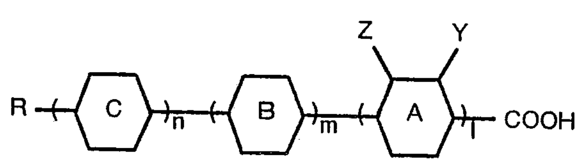

worin R, A, B, C, X, Y, Z, I, m und n so definiert sind wie im Anspruch 1, umgesetzt wird.

**Revendications**

1. Ester 2-cyano-4-halogénophénylique exprimé par la formule générale

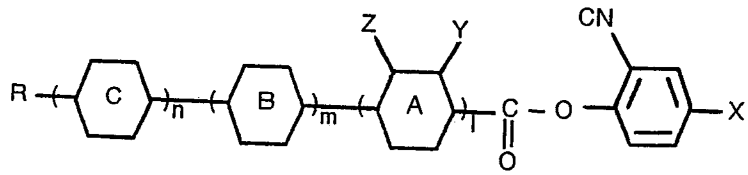

(I)

dans laquelle R représente un atome d'hydrogène ou un groupe alkyle ou un groupe alcoxy ayant 1 à 10 atomes de carbone; les cycles

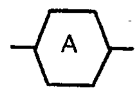
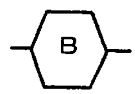
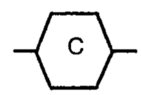

représentent chacun

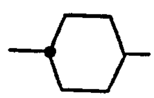

ou

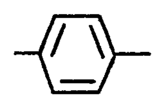

*l*, *m* et *n* représentent chacun 0 ou 1 et la somme de l + m + n est 1, 2 ou 3; X représente F ou Cl; et Y et Z représentent chacun un atome d'hydrogène ou F ou Cl.

2. Ester 2-cyano-4-halogénophénylique exprimé par la formule générale

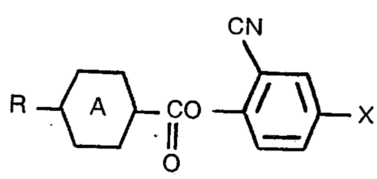

(II)

dans laquelle R, le cycle

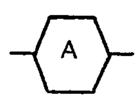

et X sont chacun comme défini dans la revendication 1.

3. Ester 2-cyano-4-halogénophénylique exprimé par la formule générale

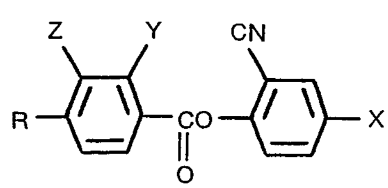

(III)

dans laquelle R, X, Y et Z sont chacun comme défini dans la revendication 1.

4. Ester 2-cyano-4-halogénophénylique exprimé par la formule générale

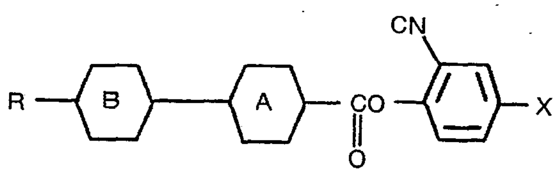

(IV)

dans laquelle R, les cycles

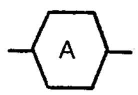

et

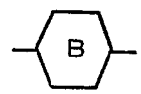

et X sont chacun comme défini dans la revendication 1.

5. Ester 2-cyano-4-halogénophénylique exprimé par la formule générale

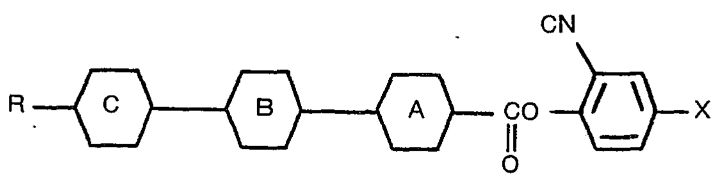

(V)

dans laquelle R, les cycles

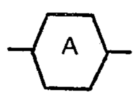

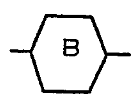

et

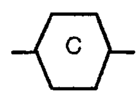

et X sont chacun comme défini dans la revendication 1.

6. Composition à cristaux liquides contenant au moins un ester selon l'une quelconque des revendications précédentes.

7. Elément d'affichage à cristaux liquides qui contient une composition selon la revendication 6.

8. Procédé pour produire un ester selon la revendication 1, qui comprend la mise en réaction d'un 2-cyano-4-halogénophénol de formule générale

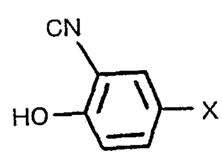

dans laquelle X est comme défini, avec un acide carboxylique de formule

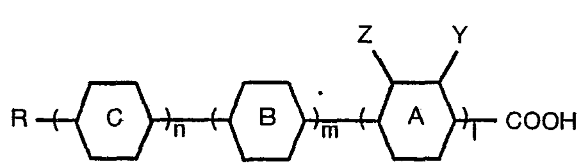

dans laquelle R, A, B, C, X, Y, Z, l, m et n sont comme défini dans la revendication 1.